(19) Europäisches Patentamt
European Patent Office
Office européen des brevets

(11) EP 0 778 270 A1

(12) EUROPEAN PATENT APPLICATION

(43) Date of publication:
11.06.1997 Bulletin 1997/24

(51) Int. Cl.[6]: C07C 409/24, C07C 407/00

(21) Application number: 96200070.9

(22) Date of filing: 12.01.1996

(84) Designated Contracting States:
AT BE CH DE DK ES FR GB GR IE IT LI LU NL PT SE

(30) Priority: 06.12.1995 EP 95203358

(71) Applicant: THE PROCTER & GAMBLE COMPANY
Cincinnati, Ohio 45202 (US)

(72) Inventors:
- Bianchetti, Giulia Ottavia
  I-00144 Roma (IT)
- Di Furia, Fulvio
  I-35128 Padova (IT)
- Scialla, Stefano
  I-00128 Roma (IT)
- Verzini, Massimo
  I-37042 Caldiero, Verona (IT)

(74) Representative: Engisch, Gautier et al
Procter & Gamble
European Technical Center N.V.
Temselaan 100
1853 Strombeek-Bever (BE)

# (54) Process for manufacturing monoperacids

(57) A process for manufacturing monopercarboxylic acids is disclosed which comprises the steps of reacting the corresponding dicarboxylic acid with a concentrated hydrogen peroxide solution in presence of a solution of a strong acid for a period of time not greater than 1 hour and extracting said monopercarboxylic acid formed.

EP 0 778 270 A1

Printed by Rank Xerox (UK) Business Services
2.14.7/3.4

## Description

### Technical field

The present invention relates to a process for manufacturing monopercarboxylic acids and their salts.

### Background

A great variety of cleaning compositions have been described in the art comprising percarboxylic acids (hereinafter also called peracids). In order to provide such cleaning compositions comprising peracids, it is common practice to incorporate peracid precursors as a source of said peracids in such cleaning compositions. Indeed, compositions containing such peracids can be made by a variety of methods employing reactions between hydrogen peroxide and the corresponding peracid precursors, e.g., the corresponding acids. However, it may be desirable for formulators to isolate the peracid from the reaction media and incorporate it as pure raw material in liquid detergent compositions.

Thus an object of the present invention is to provide peracids that may be easily incorporated in liquid detergent compositions.

Percarboxylic acids may be synthesized by different ways. For example, dipercarboxylic acids may be synthesized by reacting the corresponding dicarboxylic acids with concentrated solution of hydrogen peroxide. However the synthesis of monopercarboxylic acids from the correspondent dicarboxylic acids in presence of concentrated solution of hydrogen peroxide is difficult as dipercarboxylic acids are mostly formed.

Thus a further object of the present invention is to develop a process for manufacturing a monopercarboxylic acid from the corresponding dicarboxylic acid.

We have now found that the above objects can be efficiently met by reacting the corresponding dicarboxylic acid with a concentrated hydrogen peroxide solution in presence of an aqueous solution of a strong acid for a period of time not greater than 1 hour and extracting said monopercarboxylic acid formed. Indeed, it has been found that a higher yield of monopercarboxylic acids is achieved when limiting the contact time between the corresponding dicarboxylic acid and said concentrated hydrogen peroxide solution to less than 1 hour and then extracting said monopercarboxylic acids formed.

An advantage of the process according to the present invention is that it allows for great flexibility in formulating percarboxylic acid-containing liquid detergent compositions. Indeed, the monopercarboxylic acids obtainable according to the present invention have a higher acidity and a higher water solubility than the corresponding dipercarboxylic acids formed from the same dicarboxylic acids as the starting material and the corresponding monopercarboxylic acids formed from the corresponding monocarboxylic acids as the starting material. Thus said monopercarboxylic acids obtainable according to the present invention may be more easily incorporated in a liquid composition. In addition, monopercarboxylic acids obtainable according to the present invention with the corresponding dicarboxylic acid, as the starting material, have a lower Av.O content than the corresponding dipercarboxylic acid. The higher the Av.O content, the more hazardous the peracid, i.e., the higher the risk of explosions if it dries out.

Processes for preparing peracid solutions by reacting a hydrogen peroxide solution with an organic carboxylic acid and/or anhydride are well known in the art. European patent application number 94202608.9 discloses a process for the manufacture of aqueous compositions comprising peracids wherein said process comprises the step of forming said peracids by reacting the corresponding anhydrides with a concentrated hydrogen peroxide solution comprising at least 3 moles of said hydrogen peroxide per molar equivalent of said corresponding anhydride. Peracid concentration that is at or near its maximum is obtained in a period of less than 5 hours, and a period of not greater than 5 minutes is often sufficient in particular in a very acidic environment (pH=0 to 1) starting from the moment where anhydride is mixed with a concentrated hydrogen peroxide solution. No dicarboxylic acid is used as the starting material.

US 3 227 655 discloses that a monoperacetic acid solution can be prepared from hydrogen peroxide and acetic anhydride by reacting these reagents in an aqueous medium at a pH of about 2 to not greater than 4, in the presence of ammonium ions introduced in the form of ammonia, ammonium hydroxide or organic primary secondary or tertiary amine bases. The presence of ammonium bases is said to have a favourable effect on the bleaching process and assists particularly in the removal of the natural waxes.

WO 93/0516 discloses a process for the preparation of a dilute aqueous solution comprising a hydroxyaliphatic peroxycarboxylic acid having no more than 7 carbon atoms in which in a first step a concentrated aqueous solution of said peroxycarboxylic acid precursor, i.e. an hydroxyaliphatic carboxylic acid, is mixed with a concentrated hydrogen peroxide solution containing preferably at least 50% by weight of hydrogen peroxide in presence, if necessary of a strong acid as a catalysator, in a second step the mixture is stored until the concentration of said peroxycarboxylic acid has approached its maximum, then the mixture is diluted in water. WO 93/0516 discloses that said hydroxyaliphatic carboxylic acid may be a monocarboxylic acid, or preferably a dicarboxylic acid, or a tricarboxylic acid. Examples of said acid are lactic acid, tartaric acid, malic acid and citric acid. WO 93/0516 mentions that it is possible to attain a peracid concentration that is at or near its maximum in a period of less than 8 hours, said reaction period being at least 45 minutes.

No extraction/separation steps are disclosed. WO 93/0516 fails to make the difference between dipercarboxylic acids and monopercarboxylic acids.

EP-A- 024 219 discloses a process for the manufacture of diluted compositions of aliphatic carboxylic peracids by reacting dicarboxylic acids of from 3 to 5 carbon atoms with a concentrated solution of hydrogen peroxide (60% to 90%) in presence of a catalyst and then by diluting the resulting composition. The reaction time is of a maximum of 48 hours. No extraction/separation steps are disclosed. EP-A-024 219 fails to make the difference between dipercarboxylic acids and monopercarboxylic acids.

WO 91/13058 discloses a process for providing diluted solution of a lower aliphatic peracid, wherein hydrogen peroxide is contacted with a lower aliphatic acid each at initial high concentrations. The process is characterised in that the reaction mixture rich in lower aliphatic peracid is diluted before it has itself reached equilibrium. No extraction/separation steps are disclosed.

Processes for manufacturing peracids per se are also known. EP-A-127783 discloses a process for the desensitisation of water-insoluble aliphatic or aromatic peroxycarboxylic acids, wherein said percarboxylic acids are prepared by reacting carboxylic acids (mono or di-) with solution of hydrogen peroxide in presence of sodium sulphate at defined temperatures, optionally cooling the reacting mixture, then converting sulphuric acid present in the mixture into sodium sulphate by adding sodium hydroxide (pH=2-6) and finally the resultant reaction product is separated from the reaction mixture and dried. The reaction time between the hydrogen peroxide solution and dicarboxylic acids are of 2, 4, 6 and 8 hours (see examples). EP-A-127783 fails to make the difference between monopercarboxylic acids and dipercarboxylic acids.

BE-A-86 4135 discloses a continuous process of manufacturing a peracid involving the reaction of hydrogen peroxide with a carboxylic acid in an aqueous medium containing a mineral acid, like sulphuric acid, said hydrogen peroxide being added to the reaction mixture in several stages so as to limit everywhere the concentration in hydrogen peroxide. The peracid formed is extracted into an organic phase which contains organic solvents. The reaction and the extraction are carried out simultaneously. BE-A-86 4135 teaches that the carboxylic acids used are preferably nonsubstituted monocarboxylic acids having at least 2 but less than 6 carbon atoms. No dicarboxylic acids as starting material are expressly mentioned.

US 4 650 612 discloses a process for preparing monoperoxydicarboxylic acids by reacting the corresponding anhydrides in the presence of an anion exchange resin with hydrogen peroxide in an organic solvent stable to the peracids formed. Reaction times of 5 minutes to 5 hours, preferably 10 minutes to 1 hour are used. The reaction conditions in US 4 650 612 require the presence of organic solvent whereas in the process of the present invention an aqueous medium is used, i.e. aqueous solution of a strong acid. No dicarboxylic acids as starting materials are disclosed. No aliphatic monopercarboxylic acids are disclosed.

"Synthesis and properties of long chain aliphatic peracids" by W.E. Parker and all., J. Am. Chem. Soc. 1955, 77, 4037 discloses that long chain aliphatic peracids can be prepared directly from the parent carboxylic acids and concentrated hydrogen peroxide (50%-65%) in presence of aqueous acid sulphuric medium. The reactions are rapid at 10° to 30° C and rarely require more than 1 hour reaction time and the isolation of the product is accomplished readily by dilution of the reaction mixture with cold water followed by filtration or extraction with organic solvent (see for instance the preparation of perlauric acid). The carboxylic acids used as starting materials in this document are monocarboxylic acids, no dicarboxylic acids are disclosed as the starting material to prepare monoperacids.

None of these prior art documents discloses the preparation of monopercarboxylic acids by reacting as the starting material, the corresponding dicarboxylic acids with concentrated solution of hydrogen peroxide in presence of an aqueous solution of a strong acid, for a period not greater than 1 hour, and extracting said monopercarboxylic acid formed.

## Summary of the invention

The present invention is a process for manufacturing a monopercarboxylic acid or an alkali metal or alkaline earth metal salt thereof, comprising the steps of reacting the corresponding dicarboxylic acid with a concentrated solution of hydrogen peroxide in the presence of an aqueous solution of a strong acid for a period not greater than 1 hour and extracting said monopercarboxylic acid formed.

The present invention also encompasses an aliphatic monopercarboxylic acid, or an alkali metal, or alkaline earth metal salt thereof, obtainable by the process mentioned herein.

## Detailed description of the invention

The process according to the present invention is a process for manufacturing a monopercarboxylic acid or an alkali metal or alkaline earth metal salt thereof comprising the steps of reacting the corresponding dicarboxylic acid with a concentrated solution of hydrogen peroxide in the presence of an aqueous solution of a strong acid for a period not greater than 1 hour and extracting said monopercarboxylic acid formed.

As a first essential element, the process of the present invention requires the use of "the corresponding dicarboxylic

acid". By "the corresponding dicarboxylic acid" it is to be understood herein that in order to produce the desired monopercarboxylic acid or an alkali metal or alkaline earth metal salt thereof, the dicarboxylic acid to be used herein, as the starting material, is the dicarboxylic acid corresponding to said monopercarboxylic acid. Said dicarboxylic acid by reaction with a concentrated hydrogen peroxide solution, in the conditions of the present invention, allows to obtain said monopercarboxylic acid.

In the present invention the corresponding dicarboxylic acid which is peroxidized to obtain the desired monopercarboxylic acid may be any substituted or unsubstituted aliphatic dicarboxylic acid, or an alkali metal or alkaline earth metal salt thereof according to the following formula, or mixtures thereof :

HOOC-A-COOH

wherein A is a substituted or unsubstituted aliphatic chain of at least 1 carbon atom, preferably a substituted or unsubstituted aliphatic chain from 1 to 30 carbon atoms, more preferably from 2 to 12, and most preferably from 5 to 10.

Other suitable corresponding dicarboxylic acid which is peroxidized to obtain the monopercarboxylic acid may be any substituted or unsubstituted aromatic dicarboxylic acid, having at least 5 aromatic carbon atoms, preferably from 5 to 10, more preferably from 5 to 6, wherein at least two of said aromatic atoms are substituted by -Cn-COOH wherein n ranges from 1 to 10, preferably from 2 to 5, the remaining aromatic atoms are the same or different and are substituted by hydrogen, or by a substituted or unsubstituted aliphatic chain containing from 1 to 30 carbon atoms, preferably from 2 to 10, or by -Cn-COOH, wherein n ranges from 5 to 10, preferably from 2 to 5. Preferred substituted or unsubstituted aromatic dicarboxylic acids have the following formula:

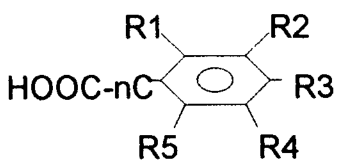

wherein n ranges from 1 to 10, preferably from 2 to 5, and wherein at least one of the groups R1, R2, R3, R4, R5 is -Cn-COOH, with n being from 1 to 10 preferably from 2 to 5, and the remaining groups are the same or different and are unsubstituted or substituted aliphatic alkyl or aromatic chain comprising at least 1 carbon atom, preferably from 1 to 30 and more preferably from 2 to 10, or -Cn-COOH with n being from 1 to 10, preferably from 2 to 5, or hydrogen.

Accordingly, examples of the corresponding dicarboxylic acids to be used according to the present invention include pimelic acid (commercially available in solid form from Aldrich), suberic acid (commercially available in solid form from Aldrich), adipic acid (commercially available in solid form from Aldrich), dodecandioic acid (commercially available in solid form from Aldrich) or alkali metal or alkaline earth metal salt thereof or mixtures thereof.

As a second essential element, the process of the present invention requires the use of a concentrated hydrogen peroxide solution, i.e. a solution comprising at least 1.1 moles hydrogen peroxide per molar equivalent of the corresponding dicarboxylic acid, preferably at least 1.5 and more preferably at least 2.

Accordingly, in the preferred embodiment, the corresponding dicarboxylic acid is mixed with a solution of hydrogen peroxide comprising from 20% to 90% by weight of said hydrogen peroxide, more preferably from 25% to 80% and most preferably from 30% to 70%.

As a third essential element, the process of the present invention requires the use of an aqueous solution of a strong acid or mixtures thereof. By strong acid it is meant herein an acid having its first pka below 3, preferably below 2 and more preferably below 1. Said aqueous solution of strong acids serves as the reaction medium and as a catalyst. Said strong acids include mineral acids and/or organic sulphonic acid. Preferred strong acids to be used herein are sulphuric acid, phosphonic acid and/or methane sulphonic acid.

In the process according to the present invention said aqueous solution of a strong acid or mixtures thereof, comprises from 90% to 98% by weight of said strong acid, more preferably from 95% to 98% and most preferably from 97% to 98%. Accordingly, the present peroxidation reaction is conducted in the acidic range at a pH below 1.5, preferably below 0.5, more preferably at acidities where the pH has no meaning anymore (i.e., below 0).

By using such concentrated aqueous solution of a strong acid it is possible to enable the peroxidation to occur at a convenient rate without the use of elevated reaction temperature. The temperature at which the present peroxidation reaction is conducted also depends on the concentration of the solution of hydrogen peroxide used. If the solution of hydrogen peroxide is very concentrated (e.g., 70%), the reaction is preferably conducted around 20°C. If it is less concentrated (e.g., 36%), the reaction is preferably conducted at room temperature, e.g., 25°C or higher. For convenience, coupled with safety considerations, the reaction temperature is maintained in many embodiments in the range that is from 0°C to 40°C.

As a fourth essential element, the process of the present invention for manufacturing monopercarboxylic acid requires a reaction time between the concentrated solution of hydrogen peroxide and the corresponding dicarboxylic acid in presence of a solution of a strong acid of not more than 1 hour, preferably of from 40 minutes to 60 minutes, more preferably of from 50 minutes to 55 minutes. It has now been found that a higher yield of monopercarboxylic acid is obtained by limiting the reaction time between the concentrated solution of hydrogen peroxide and the corresponding dicarboxylic acid in presence of a solution of a strong acid to less than 1 hour and then extracting said monopercarboxylic acid formed. A reaction time of more than 1 hour is in favour of the formation of dipercarboxylic acids.

In the process of the present invention the reaction step as described above is followed by an extraction step wherein the monopercarboxylic acid formed is separated off from the reaction mixture. By "extraction step" it is meant herein any separation step that allows to isolate said monopercarboxylic acid formed from the reaction mixture.

Thus extracting according to the present invention includes any separation method known in the art. Accordingly, extraction may include induced precipitation by $(NH_4)_2SO_4$, filtration, centrifugation, extraction by organic solvents and the like.

Preferred herein the extraction step includes filtering away the dipercarboxylic acid that may have formed from the remaining reaction mixture and isolating said monopercarboxylic acid by extraction with organic solvents.

Organic solvents suitable to be used herein include ethyl ether, ethyl acetate, chlorinated hydrocarbons such as $CH_2Cl_2$, $CHCl_3$.

An advantage associated with the process of the present invention is that a fast and easy preparation with a high yield of monopercarboxylic acids is obtained. A further advantage associated with the process of the present invention is that the above benefits are obtained without the use of elevated reaction temperatures.

By "yield" it is to be understood herein the percentage of monopercarboxylic acid obtained calculated with respect to the corresponding dicarboxylic acid. The following equation is applied to calculate said yield:

(monopercarboxylic acid concentration / corresponding dicarboxylic acid concentration ) * 100 = % yield

In said equation the concentrations are expressed in mole/liter. Indeed, it has surprisingly been found that a higher yield of monopercarboxylic acid is provided with the present process starting from a given amount of the corresponding dicarboxylic acid and reacting it in presence of an aqueous solution of a strong acid with a concentrated hydrogen peroxide solution for a period of time not greater than one hour before being extracted, as compared to a similar process wherein the corresponding dicarboxylic acid is left into contact with said concentrated solution of hydrogen peroxide for longer period of time before being extracted.

The present invention also encompasses aliphatic monopercarboxylic acids or alkali metal or alkaline earth metal salts thereof obtainable according to the process of the present invention. Said aliphatic monopercarboxylic acids according to the present invention obtainable from the corresponding dicarboxylic acid have higher acidity and higher water solubility than the corresponding dipercarboxylic acids obtained from the same corresponding dicarboxylic acids. The aliphatic monopercarboxylic acids of the present invention maintain a carboxylic group which is more acid than the percarboxylic one. Also the hydrogen bonding of monopercarboxylic acids according to the present invention with water molecules is more effective than for dipercarboxylic acids obtained using the same dicarboxylic acids as the starting material. Accordingly, as the aliphatic monopercarboxylic acids of the present invention are more soluble than the corresponding dipercarboxylic acids and the corresponding monoperacids formed from monocarboxylic acids as the starting material, their incorporation in liquid aqueous detergent compositions is easier. Indeed, said aliphatic monopercarboxylic acids obtainable according to the process of the present invention may be incorporated in laundry detergent, in laundry pretreaters, in laundry additives, in dishwashing compositions, in carpet cleaners, in toilet bowl cleaners, in hard surface cleaners and in liquid disinfectants.

In addition monopercarboxylic acids obtainable according to the process of the present invention starting from the correspondent dicarboxylic acid as the starting material have a lower Av.O content that the corresponding dipercarboxylic acid obtainable from the same starting material. Av.O is defined as the number of peracidic oxygens x 16/MW of the peracid. The higher the Av.O content, the more hazardous the peracid, i.e., the higher the risk of explosions if it dries out. The presence of one carboxylic group left may be used for making other peroxydic derivatives such as esters or anhydrides and for anchoring the monoperacids to polymeric matrixes.

The aliphatic monopercarboxylic acid obtainable according to the present process have the following formula:

HOOC-A-COOOH

wherein A is a substituted or unsubstituted aliphatic chain of at least 1 carbon atom, preferably a substituted or unsubstituted aliphatic chain from 1 to 30 carbon atoms, more preferably from 2 to 12, and most preferably from 5 to 10.

Aliphatic monopercarboxylic acids obtainable according to the process of the present invention can be identified by measuring the monoperacid available oxygen (often abbreviated to Avox). Test method to evaluate peracid Avox is done via chromatography (see F. Di Furia et. alt., Gas-liquid chromatography method for determination of peracids, Analyst,

vol. 109, August 1984, p. 985-987; or ibidem vol. 113, May 1988, p. 793-795).

The present invention will be further illustrated by the following examples.

Examples

Monopercarboxylic acids such as monoperpimelic or monopersuberic were obtained by carrying out the following method. 0.02 moles of the corresponding dicarboxylic acid, i.e., pimelic acid or suberic acid were solubilized in 18 grs of a concentrated aqueous solution of sulphuric acid (95% by weight). Then said solution was contacted under stirring with a concentrated solution of hydrogen peroxide (70% by weight) that was added drop by drop up to 100% excess with respect to pimelic acid. After the addition of said concentrated solution of hydrogen peroxide, the reaction mixture was stirred at room temperature (about 20-25°C) for 50 minutes. Thereafter an extraction was carried out by washing the reaction mixture with a cold (0 °C) water solution of ammonium sulfate (35 grs in 100 grs of water), filtering away the dipercarboxylic acid (i.e., diperpimelic, dipersuberic) that may have formed and isolating 1.69 grs of monoperpimelic acid and 1.24 grs of monopersuberic acid from the remaining mixture by extraction with ethyl ether. The yield of monoperpimelic acid was of 48%. The yield of monopersuberic was 33%.

## Claims

1. A process for manufacturing a monopercarboxylic acid or an alkali metal or alkaline earth metal salt thereof comprising the steps of reacting the corresponding dicarboxylic acid with a concentrated solution of hydrogen peroxide in the presence of an aqueous solution of a strong acid for a period not greater than 1 hour and extracting said monopercarboxylic acid formed.

2. A process according to claim 1 wherein said solution of hydrogen peroxide comprises at least 1.1 moles of said hydrogen peroxide per molar equivalent of said corresponding dicarboxylic acid, preferably at least 1.5, and more preferably at least 2.

3. A process according to any of the preceding claims wherein said solution of hydrogen peroxide comprises from 20% to 90% by weight of said hydrogen peroxide, preferably of from 25% to 80% and more preferably of from 30% to 70%.

4. A process according to any of the preceding claims wherein said dicarboxylic acid is a substituted or unsubstituted aliphatic dicarboxylic acid, or an alkali metal or alkaline earth metal salt thereof of the following formula, or mixtures thereof :

   HOOC-A-COOH

   wherein A is a substituted or unsubstituted aliphatic chain of at least 1 carbon atom, preferably a substituted or unsubstituted aliphatic chain from 1 to 30 carbon atoms, more preferably from 2 to 12 carbon atoms, and most preferably from 5 to 10 carbon atoms, a substituted or unsubstituted aromatic dicarboxylic acid, having at least 5 aromatic carbon atoms, preferably from 5 to 10, more preferably from 5 to 6, wherein at least two of said aromatic atoms are substituted by -Cn-COOH, wherein n ranges from 1 to 10, preferably from 2 to 5, the remaining aromatic atoms are the same or different and are substituted by hydrogen, or by a substituted or unsubstituted aliphatic chain containing from 1 to 30 carbon atoms, preferably from 2 to 10, or by -Cn-COOH, wherein n ranges from 1 to 10, preferably from 2 to 5, or mixtures thereof.

5. A process according to any of the preceding claims wherein said dicarboxylic acid is pimelic acid, suberic acid, adipic acid, dodecandioic acid, or an alkali metal or alkaline earth metal salt thereof or mixtures thereof.

6. A process according to any of the preceding claims wherein said strong acid is an acid having a pka below 3, preferably below 2 and more preferably is sulphuric acid, phosphonic acid and/or methane sulphonic acid.

7. A process according to any of the preceding claims wherein said aqueous solution of strong acid comprises from 90% to 98% by weight of strong acid, preferably from 95% to 98% and more preferably from 97% to 98%.

8. A process according to any of the preceding claims wherein said concentrated solution of hydrogen peroxide is reacted with said corresponding dicarboxylic acid in the presence of an aqueous solution of a strong acid for a period of from 40 to 60 minutes, preferably of from 50 to 55 minutes before extracting said monopercarboxylic acid.

9. A process according to any of the preceding claims wherein said extraction comprises filtering and extraction with an organic solvent, preferably ethyl acetate and/or chlorinated hydrocarbons.

10. An aliphatic monopercarboxylic acid or an alkali metal or alkaline earth metal salt thereof obtainable by a process according to any of the preceding claims.

European Patent Office

# EUROPEAN SEARCH REPORT

Application Number
EP 96 20 0070

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
| --- | --- | --- | --- |
| D,A | DE-A-34 26 792 (DEGUSSA)<br>* the whole document * | 1 | C07C409/24<br>C07C407/00 |
| D,A | FR-A-2 381 752 (PROPYLOX SA)<br>* the whole document * | 1 | |

TECHNICAL FIELDS SEARCHED (Int.Cl.6)

C07C

The present search report has been drawn up for all claims

| Place of search | Date of completion of the search | Examiner |
| --- | --- | --- |
| THE HAGUE | 17 April 1996 | Bonnevalle, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)